# EUROPEAN PATENT SPECIFICATION

(11) **EP 2 010 218 B1**
(45) Date of publication and mention of the grant of the patent: **10.10.2018**
(21) Application number: 07754798.2
(22) Date of filing: 04.04.2007
(51) Int. Cl.: A61P 3/02, A61K 31/22

(54) **COMPOSITIONS COMPRISING PYRUVATE ALKYL ESTERS AND USES THEREOF**
ZUSAMMENSETZUNGEN MIT PYRUVAT-ALKYLESTERN UND IHRE ANWENDUNGEN
COMPOSITIONS COMPRENANT DES ESTERS DE PYRUVATE D'ALKYLE ET UTILISATIONS

(30) Priority: 12.04.2006 US 744739 P
(43) Date of publication of application: 07.01.2009
(73) Proprietor: Alexander, Ranya L., San Diego, CA 92130 (US)
(72) Inventor: Alexander, Ranya L., San Diego, CA 92130 (US)
(74) Representative: Viering, Jentschura & Partner mbB Patent- und Rechtsanwälte
(86) International application number: PCT/US2007/008334
(87) International publication number: WO 2007/120544

(56) References cited:
- WO-A1-97/34856
- WO-A1-98/04254
- WO-A1-03/088956
- WO-A2-2006/015232
- WO-A2-2006/108679
- US-A- 5 294 641
- US-A- 5 480 909
- US-A- 5 621 006
- US-A1- 2003 073 743
- YANG R ET AL: "ETHYL PYRUVATE MODULATES INFLAMMATORY GENE EXPRESSION IN MICE SUBJECTED TO HEMORRHAGIC SHOCK" AMERICAN JOURNAL OF PHYSIOLOGY: GASTROINTESTINAL AND LIVER PHYSIOLOGY, AMERICAN PHYSIOLOGICAL SOCIETY, US, vol. 283, 1 January 2002 (2002-01-01), pages G212-G221, XP008019310 ISSN: 0193-1857

## Description

### BACKGROUND OF THE INVENTION

The present invention relates generally to the field of compositions for oral supplementation for energy enhancement, physical performance enhancement, or mood enhancement. Specifically, the present invention relates to oral compositions comprising ethylpyruvate for the after-mentioned uses.

Ethylpyruvate occurs naturally in a variety of products, including beer. The compound is generally recognized as safe by the United State Food and Drug Administration as a synthetic flavoring substance. "Synthetic flavoring substance" refers to substances and adjuvants which may be safely used in food in accordance with the following conditions: (a) they are used in the minimum quantity required to produce their intended effect, and otherwise in accordance with all the principles of good manufacturing practice; and (b) they consist of one or more of the compounds listed in 21 C.F.R. § 172.515, used alone or in combination with flavoring substances and adjuvants generally recognized as safe in food, prior-sanctioned for such use, or regulated by an appropriate section of 21 C.F.R. § 172.

U.S. 4,158,057 reports a medical method of preventing excessive accumulation of fatty deposits in the liver of a mammal due to ingestion of ethanol, which method includes administering orally to the mammal a mixture of pyruvate and dihydroxyacetone, optionally riboflavin.

U.S. 5,294,641 reports a medical method for increasing the cardiac output of a human without concurrently increasing the cardiac oxygen demand of the human which comprises includes feeding the human, orally or intravenously, pyruvate, optionally as an organic ester.

U.S. 5,480,909 reports a medical method for inhibiting free-radical generation and concurrently scavenging internally generated-free radicals in a mammal by administering to the mammal pyruvate, or a pharmaceutically acceptable ester thereof.

U.S. 5,801,198 reports a medical method for retarding loss of morphology in the bowel of a mammal experiencing ischemic bowel by introducing pyruvate, or organic ester thereof, enterally or parenterally into the mammal prior to and during the ischemic bowel or during reperfusion.

U.S. 6,846,842 describes a medical method for treating a mammal suffering from ischemia or reperfusion injury by administering an ester of a 2 ketoalkanoic acid selected from the group consisting of ethyl pyruvate, propyl pyruvate, butyl pyruvate, carboxymethyl pyruvate, acetoxymethyl pyruvate, carbethoxymethyl pyruvate and ethoxymethyl pyruvate in a pharmaceutically-acceptable carrier, wherein the carrier further comprises an organic or inorganic cation.

Additional uses for pyruvate administration have been reported in, e.g.,: U.S. 4,874,790 (medical use of pyruvate for diabetes treatment; U.S. 4,812,479, 4,548,937 and 4,351,835 (medical use in retarding weight gain); U.S. 4,415,576 (medical use to increase body protein concentration in a mammal; U.S. 4,315,835 (use for extending athletic endurance; U.S. 5,134,162 (medical use for retarding cholesterol increase; and U.S. App. Ser. No. 08/194.857. filed Feb. 14, 1994 (medical use for inhibiting growth in spread of malignancy and retarding DNA breaks).

Compositions comprising ethylpyruvate for use as a food or dietary supplement have been available in the prior art (WO 2006/108679 A2; WO 03/088956 A1; US 5621996 A). However, there still remains a considerable need for such compositions that can be used for ingestion by a human as a food or dietary supplement in order to enhance energy, physical performance, or mood in the human.

### SUMMARY OF THE INVENTION

In accordance with the present invention, a variety of benefits as described herein accompany the ingestion of ethylpyruvate by a human.

The present invention provides a composition for ingestion by a human as a food, or dietary supplement, which composition comprises ethylpyruvate, wherein the ethylpyruvate is present at greater than 0.01 weight-percent, for use for energy enhancement, physical performance enhancement, or mood enhancement in said human.

Without wishing to be bound by any specific scientific theory, it is believed that pyruvate esters, and in particular ethylpyruvate, are sufficiently lipophilic to be taken up by cells at a faster rate than equimolar amounts of pyruvic acid *per se* or any tautomeric or charged forms thereof. Accordingly, ethylpyruvate in the compositions of the present invention serves as a carrier for intracellular pyruvate delivery made bioavailable after non-specific ester solvolysis by ubiquitous cytosolic carboxyesterases. Pyruvate oxidation connects glycolysis to the tricarboxylic acid cycle whereby pyruvate molecules formed during glycolysis are transported from the cytoplasm through the mitochondrial membranes into the mitochondrial matrix, wherein the pyruvate dehydrogenase complex catalyzes known biochemical reactions which result in the production of acetyl-CoA. Accordingly, direct provision of pyruvic acid circumvents the need for production of pyruvic acid via glycolysis for cellular energy.

"Ingestion" refers in the context of compositions and methods of the present invention to the taking of a composition into the body via the alimentary canal.

"Dietary supplement" refers to a product as defined under the United States Dietary Supplement Health and Education Act of 1994, which product is intended to supplement the diet and bears or contains one or more of a vitamin, a mineral; an herb or other botanical (excluding tobacco), an amino acid, a dietary substance for use by man to supplement the diet by increasing the total dietary intake, or a concentrate, metabolite, constituent, extract or combination of any of these components. A dietary supplement is intended for ingestion in pill, capsule, tablet, powder or liquid form, and is not represented for use as a conventional food or as the sole item of a meal or diet labeled as a "dietary supplement." The terms "oral supplement" and "dietary supplement" are.used interchangeably herein.

"Weight-percent," the symbol "wt-%" and equivalent terms refer to the ratio of the mass of a specified component of a composition to the total mass of the composition, expressed as a percentage. Accordingly, 0.1 weight-percent is equivalent to 1.0 gm/kg (i.e., 1000 parts per million), 0.01 weight-percent is equivalent to 0.1 gm/kg (i.e., 100 parts per million), and so forth.

The term "ethylpyruvate" contemplates all tautomeric and charged forms of the compound according to any of Formulae I-III having R¹ equal to ethyl. In some embodiments, the nutritional content afforded by ethylpyruvate as an oral supplement accounts for 0.1-50% of the daily nutritional needs of the subject. In some embodiments, the nutritional content afforded is within another range, e.g., 0.1-40%, 0.1-30%, 0.1-20%, 0.1-10%, 0.1-5%, 0.1-1%.

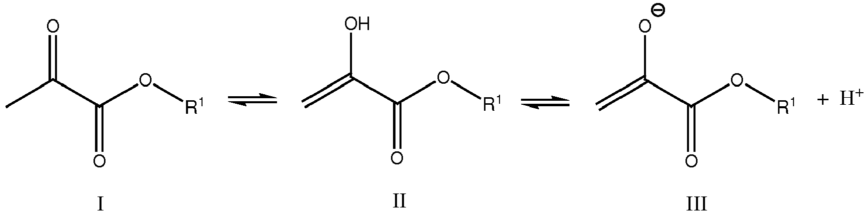

"Energy enhancement" refers to a subjective feeling by a human subject that the strength, stamina and/or inclination to undertake a task has been increased by ingestion of a composition of the invention. An energy enhancement may be accompanied by feelings that additional need for energy is not required. For example without limitation, energy enhancement may accompany a feeling by a human subject that additional stimulation e.g., ingesting a caffeine containing food or beverage, is not necessary in the contemplation of a task. Thus, by ingesting an effective amount of a composition of the invention, energy enhancement is achieved, which may be associated with reduced intake of stimulants such as caffeine.

The term "effective amount" and like terms indicate that the amount of material is effective to achieve the desired result, for example without limitation, energy enhancement in a human. Accordingly, an effective amount of a composition according to the present invention may contemplate a variety of ranges of amount of ethylpyruvate, depending on the human subject, including for example without limitation, 100 mg to 100 gm, 100 mg to 200 gm, 100 mg to 500 gm, and specific amounts therein, e.g.: about 100 mg, 200 mg, 500 mg, 1 gm, 2, gm, 3 gm, 4 gm, 5 gm, 10 gm, 15 gm, 20 gm, 30 gm, 40 gm, 50 gm, 100 gm, 200 gm, 300 gm, 400 gm or even 500 gm. These amounts can be delivered by multiple administrations over a period of time.

"Physical performance" refers to objective indicia of physical activity, as well known in the art, and to a subjective feeling or belief by a human subject that greater physical ability attends the ingestion of a composition according to the present invention. "Enhancement of physical performance" refers to both an objective increase in, for example and without limitation, strength and stamina of a human, as judged by physiological methods well known in the art, and additional to a subjective increase in feelings of well-being and potential to undertake a physical task.

"Mood" and like terms in the context of human feelings refer to a subjective self-assessment of a human, for example without limitation, feelings of being tired, normal, energetic, excited, and the like, all readily understood. Accordingly, "mood enhancement" and like terms refer to an improvement (e.g., more elevated, typically happier or more satisfied) in mood following ingestion of the composition.

### DETAILED DESCRIPTION OF THE PREFERRED EMBODIMENTS

In certain embodiments of compositions according to the present invention, the amount of ethylpyruvate is present in the range greater than 0.01 to 30 weight-percent. In other embodiments, the range is greater than 0.01 to about 30 weight-percent. "About" in the context of numeric values and ranges indicates the nominal value +/- 10%. In certain embodiments, the amount of ethylpyruvate is present in the following approximate ranges (i.e., +/- 10%): 0.02-0.05, 0.05-0.1, 0.1-1, 1-2, 2-5, 5-10, 10-20, or 20-30 weight-percent. In certain embodiments, the amount of ethylpyruvate is present in specific amounts, e.g., about 0.02, 0.05, 0.1, 1, 2, 3, 4, 5, 6, 7, 8, 9, 10, 15, 20, 25 or even 30 weight-percent.

In certain embodiments, the present invention provides compositions for oral ingestion comprising ethylpyruvate, wherein the composition lacks sodium or calcium. In certain embodiments, the present invention provides compositions for oral ingestion comprising ethylpyruvate, wherein the composition further comprises sodium, calcium, and/or magnesium.

In certain embodiments, the present invention provides compositions for oral ingestion comprising ethylpyruvate, wherein the compositions are in the form of food. As used herein, a "food" is a nutritious solid, semi-solid, liquid, food ingredient or food additive. "Semi-liquid" refers in the context of food to an otherwise solid component dispersed in a liquid milieu, e.g. without limitation, cereal in milk. A food bar or candy bar comprising ethylpyruvate is an exemplary solid food composition of the invention.

In certain embodiments, the compositions provided by the invention are foods or dietary supplements in the form of a beverage. In certain embodiments, the compositions are foods. In certain embodiments, the compositions are dietary supplements.

A liquid or beverage composition for oral ingestion can be taken cold (e.g., with ice), at room temperature, or after heating to a convenient temperature (e.g., 80-120 degrees F) depending on the needs of the human.

In certain embodiments, compositions provided by the present invention further comprise one or more components selected from the group consisting of flavorings, colorings, non-carbohydrate sweeteners, vitamins, electrolytes, Coenzyme Q₁₀, aloe, minerals, fulvic acid, mushrooms, dissolved effervescent gas, amino acids, carbohydrates, stimulants, proteins, herbs, essential oils and extracts.

In certain embodiments of methods contemplated by the invention, a single ingestion of a composition provided herein is sufficient to elicit a beneficial response. For example without limitation, increase in physical performance and enhancement of mood, can be observed upon ingestion of a single portion of a composition of the invention. In certain embodiments, the single portion provides the nutritional content equivalent to 0.1-50% of the daily nutritional needs of the subject. In some embodiments, the nutritional content afforded is within another range, e.g., 0.1-40%, 0.1-30%, 0.1-20%, 0.1-10%, 0.1-5%. Advantageously, rapid onset of beneficial effects, for example without limitation, within 1, 2, 3, 4, 5, 10, 15, or as little as 20 min, is afforded in methods contemplating oral ingestion of ethylpyruvate according to the invention. As used herein, a single portion of a composition of the invention is generally a volume of fluid or solid than can be ingested in one sitting, which sitting may last from less than a minute to several minutes and generally up to less than an hour. Exemplary single portions can include 1-4 oz solid bar or an 8-32 oz drink.

In certain embodiments of methods contemplated by the invention, repeated ingestion of a composition provided herein facilitates a beneficial response. In certain embodiments, the portions contemplated for repeated ingestion provide nutritional content equivalent to 0.1-50% of the daily nutritional needs of the subject. In some embodiments, the nutritional content afforded is within another range, e.g., 0.1-40%, 0.1-30%, 0.1-20%, 0.1-10%, 0.1-5%.

### Example 1 - Stability

It has been observed that ethylpyruvate in solution demonstrates higher stability at room temperature (e.g., about 50 to 90 degrees F) than does the methyl ester of pyruvic acid, or corresponding tautomeric and/or charged forms thereof, as judged by appearance (e.g., color) and taste. Without wishing to be bound by theory, it is believed that methyl esters of pyruvic acid are more susceptible to hydrolysis in aqueous solution than are corresponding ethyl esters, which hydrolysis results in the production of pyruvic acid. Pyruvic acid, and the anion thereof, can then react by mechanisms well known in the art, to form a plethora of higher molecular compounds. Accordingly, the higher stability of ethylpyruvate results in longer shelf life for compositions of the present invention, as compared with corresponding compositions employing the methyl ester of pyruvic acid.

### Example 2 - Taste Assessment

A panel of 10 individuals ingested a liquid composition (about 30 ml) at room temperature comprising ethylpyruvate (about 1 weight-percent) to provide a taste assessment. The panel was additionally provided a corresponding liquid composition of methylpyruvate (i.e., methyl ester of pyruvic acid) for comparative tasting. Each of the individuals judged the methylpyruvate solution to be "sour." In contrast, each of the individuals judged the ethylpyruvate solution to have the taste associated with Granny Smith apples. Without wishing to be bound by theory, it is believed that the sour taste associated with methypyruvate results from hydrolysis of methylpyruvate within the sample to pyruvic acid, with the associated source taste of an acid. In contrast, the taste associated with ethylpyruvate appears to reflect a lack of hydrolysis to pyruvic acid in the sample.

### Example 3 - Gastric Reflux

In the taste assessment experiment (Example 2), gastric reflux (i.e., "heartburn") was a considerable problem (i.e., 5 of 10 individuals reporting such) with methylpyruvate, but none reporting similar reaction with ethylpyruvate. Without wishing to be bound by any theory, it is believed that the free pyruvic acid in the methylpyruvate sample, as a result of ester hydrolysis prior to ingestion, resulted in pyruvic acid eliciting a gastric reflux reaction.

### Example 4 - Mood enhancement

In the taste assessment experiment (Example 2), mood was assessed before and after ingestion of the liquid composition comprising ethylpyruvate. Each of the individuals reported an increase in feelings of well-being and energy after ingesting the composition. This mood enhancement commenced at between 5 and 15 minutes and lasted over 6 hours. In one case, the individual stated that the usual afternoon cup of coffee would not be necessary that day due to the ingestion of the composition.

### Example 5 - Exemplary Compositions

Exemplary compositions of the present invention could include a carbonated beverage comprising ethylpyruvate, optionally added vitamins and minerals, to provide a refreshing alternative to soft drinks. Methods for formulation of such carbonated beverages are well known in the art. Exemplary vitamin and mineral components include vitamins B3, B6, B12; exemplary minerals include zinc, sodium, magnesium, and calcium.

Further exemplary compositions could include candy bars or energy bars comprising ethylpyruvate. Methods of manufacture of candy bars are well known in the art (see e.g., U.S. 4,491,597). In candy bars contemplated by the present invention, ethylpyruvate can be suspended or dissolved in a candy matrix forming the interior of the candy bar, or suspended or dissolved in an optional coating, as known in the art.

Further exemplary compositions could include suspensions or solutions of ethylpyruvate in combination with shaved, granulated, crushed, or pulverized ice to afford, for example without limitation, "snow cones" or "slush" drinks comprising ethylpyruvate. As is well known, snow cones are made by depositing flaked ice into a cone shaped cup and applying a flavoring syrup thereon (see U.S. 4,174,742). As further well known in the art, slush drinks comprise ice and flavorings and are typically dispensed at the point of sale with specialized machinery, well known in the art. Accordingly, the present invention contemplates snow cones or slush drinks to which ethylpyruvate has been added.

Further exemplary compositions could include chewing gums or soups to which ethylpyruvate has been added.

### Example 6 - Post-surgical Oral Ingestion of Ethylpyruvate

Patient response to ingestion of a composition according to the invention was observed in a patient following surgery and radiation therapy for gastrointestinal carcinoma. The patient had developed gastrointestinal inflammation and could not tolerate food by mouth for several days post-operative. Furthermore, bowel movements were frequent, watery and bloody, and were accompanied by lower abdominal pain. The patient self administered oral ethylpyruvate (about 3x500 mL per day daily for 1 week of an ethylpyruvate solution in commercial bottled spring water at 0.06 weight-percent ethylpyruvate). Within 3 hours of the initial dosing, persistent pain, hyperactive bowel and diarrhea resolved. The patient additionally reported an improved mood following oral ingestion of ethylpyruvate.

### Example 7 - Athlete Oral Ingestion of Ethylpyruvate

A group of 12 athletes (19-22 years of age) self-administered oral ethylpyruvate in solution (4x500mL ingested per day of a solution of about 0.06 weight-percent ethylpyruvate in water) before and during daily workouts (approximately 2 hours duration) for four days. The athletes having ingested oral ethylpyruvate reported less fatigue at the end of each workout, and similar favorable reports continued each day for the four days of practice. Each subject additionally reported better performance in high-activity training regimens (e.g., "wind sprints" as known in the art) relative to performance reported in practice sessions prior to oral ingestion of ethylpyruvate.

All patents and other references cited in the specification are indicative of the level of skill of those skilled in the art to which the invention pertains.

## Claims

1. A composition for ingestion by a human as a food, or dietary supplement, said composition comprising greater than 0.01 weight-percent ethylpyruvate, for use for energy enhancement, physical performance enhancement, or mood enhancement in said human.

2. The composition for use according to Claim 1, wherein said ethylpyruvate is present in the range >0.01 to 30 weight-percent.

3. The composition for use according to claim 1, wherein said ethylpyruvate is present in the range 0.05-0.1 weight-percent in said composition.

4. The composition for use according to claim 1, wherein said ethylpyruvate is present in the range 0.1-1 weight-percent in said composition.

5. The composition for use according to claim 1, wherein said ethylpyruvate is present in the range 1-2 weight-percent in said composition.

6. The composition for use according to any one of claims 1-5, wherein said food or dietary supplement is a solid bar.

7. The composition for use according to any one of claims 1-6, in which the composition further comprises one or more other components selected from the group consisting of flavorings, colorings, non-carbohydrate sweeteners, vitamins, electrolytes, Coenzyme Q10, aloe, minerals, fulvic acid, mushrooms, dissolved effervescent gas, amino acids, carbohydrates, stimulants, proteins, herbs, essential oils and extracts.

## Patentansprüche

1. Eine Zusammensetzung zur Einnahme als ein Nahrungsmittel oder Nahrungsergänzungsmittel durch einen Menschen, wobei die Zusammensetzung mehr als 0,01 Gewichtsprozent Ethylpyruvat umfasst, zur Verwendung zur Energiesteigerung, physischen Leistungssteigerung oder Stimmungssteigerung in dem Menschen.

2. Die Zusammensetzung zur Verwendung gemäß Anspruch 1, wobei das Ethylpyruvat im Bereich von >0,01 bis 30 Gewichtsprozent vorliegt.

3. Die Zusammensetzung zur Verwendung gemäß Anspruch 1, wobei das Ethylpyruvat im Bereich von 0,05-0,1 Gewichtsprozent in der Zusammensetzung vorliegt.

4. Die Zusammensetzung zur Verwendung gemäß Anspruch 1, wobei das Ethylpyruvat im Bereich von 0,1-1 Gewichtsprozent in der Zusammensetzung vorliegt.

5. Die Zusammensetzung zur Verwendung gemäß Anspruch 1, wobei das Ethylpyruvat im Bereich von 1-2 Gewichtsprozent in der Zusammensetzung vorliegt.

6. Die Zusammensetzung zur Verwendung gemäß einem der Ansprüche 1-5, wobei das Nahrungsmittel oder Nahrungsergänzungsmittel ein fester Riegel ist.

7. Die Zusammensetzung zur Verwendung gemäß einem der Ansprüche 1-6, wobei die Zusammensetzung ferner ein oder mehrere andere Komponenten, ausgewählt aus der Gruppe bestehend aus Geschmacksstoffen, Farbstoffen, Nicht-Kohlenhydrat-Süßstoffen, Vitaminen, Elektrolyten, Coenzym Q10, Aloe, Mineralien, Fulvinsäure, Pilzen, gelöstem sprudelndem Gas, Aminosäuren, Kohlenhydraten, Stimulanzien, Proteinen, Kräutern, ätherischen Ölen und Extrakten, umfasst.

## Revendications

1. Composition pour une ingestion par un être humain en tant qu'aliment ou complément alimentaire, ladite composition comprenant plus de 0,01 % en poids de pyruvate d'éthyle, pour une utilisation pour l'amélioration de l'énergie, l'amélioration des performances physiques ou l'amélioration de l'humeur chez ledit être humain.

2. Composition pour une utilisation selon la revendication 1, dans laquelle ledit pyruvate d'éthyle est présent dans la plage > 0,01 à 30 % en poids.

3. Composition pour une utilisation selon la revendication 1, dans laquelle ledit pyruvate d'éthyle est présent dans la plage de 0,05 à 0,1 % en poids dans ladite composition.

4. Composition pour une utilisation selon la revendication 1, dans laquelle ledit pyruvate d'éthyle est présent dans la plage de 0,1 à 1 % en poids dans ladite composition.

5. Composition pour une utilisation selon la revendication 1, dans laquelle ledit pyruvate d'éthyle est présent dans la plage de 1 à 2 % en poids dans ladite composition.

6. Composition pour une utilisation selon l'une quelconque des revendications 1 à 5, dans laquelle ledit aliment ou complément alimentaire est une barre solide.

7. Composition pour une utilisation selon l'une quelconque des revendications 1 à 6, dans laquelle la composition comprend en outre un ou plusieurs autres composants choisis dans le groupe constitué par les agents aromatisants, les agents colorants, les édulcorants non glucidiques, les vitamines, les électrolytes, la coenzyme Q10, l'aloé, les minéraux, l'acide fulvique, les champignons, les gaz effervescents dissous, les acides aminés, les glucides, les stimulants, les protéines, les herbes, les huiles essentielles et les extraits essentiels.
